# EUROPEAN PATENT SPECIFICATION

(11) **EP 1 727 890 B1**
(45) Date of publication and mention of the grant of the patent: **14.05.2008**
(21) Application number: 05728073.7
(22) Date of filing: 24.03.2005
(51) Int. Cl.: C12N 1/19

(54) **MUTATED XYLOSE REDUCTASE IN XYLOSE-FERMENTATION BY S. CEREVISIAE**
MUTIERTE XYLOSEREDUKTASE BEI DER XYLOSEVERGÄRUNG DURCH S. CEREVISIAE
XYLOSE REDUCTASE MUTEE LORS DE LA FERMENTATION DU XYLOSE PAR S. CEREVISIAE

(30) Priority: 26.03.2004 SE 0400816
(43) Date of publication of application: 06.12.2006
(73) Proprietor: Forskarpatent i Syd AB, 223 70 Lund (SE)
(72) Inventor: GORWA-GRAUSLUND, Marie-Françoise, SE-218 53 Klagshamn (SE); JEPPSON, Marie, S-245 32 Staffanstorp (SE)
(74) Representative: Valea AB
(86) International application number: PCT/SE2005/000453
(87) International publication number: WO 2005/093041

(56) References cited:
- KOSTRZYNSKA M. ET AL: 'Mutational analysis of the role of the conserved lysine-270 in the Oichia stipitis xylose reductase' FEMS MICROBILOGY LETTERS vol. 159, 1998, pages 107 - 112, XP002991637
- TRAFF-BJERRE K.L. ET AL: 'Endogenous NADPH-dependent aldose reductase activity influences product formation during xylose consumption in recombinant Saccharomyces cerevisiae' YEAST vol. 21, no. 2, 2004, pages 141 - 150, XP002991638
- JEPPSON M. ET AL: 'Effect of enhanced xylose reductase activity on xylose consumption and product distribution in xylose-fermenting recombinant Saccharomyces cerevisiae' FEMS YEAST RESEARCH vol. 3, 2003, pages 167 - 175, XP002991639
- ELIASSON A. ET AL: 'Anaerobic Xylose Fermentation by Recombinant Saccharomyces cerevisiae Carrying XYL1, XYL2, and XKS1 in Mineral Medium Chemostat Cultures' APPLIED AND ENVIRONMENTAL MICROBIOLOGY August 2000, pages 3381 - 3386, XP000973771

## Description

### Technical field

The present invention relates to a new Saccharomyces cerevisiae strain having improved properties of fermenting xylose to ethanol.

### Background of the invention

Ethanol is efficiently produced from hexoses by *Saccharomyces cerevisiae,* but recombinant *S. cerevisiae* strains capable of xylose utilisation are needed to expand the substrate range to lignocellulosic hydrolysate. By integration of the *Pichia stipitis XYL1* and *XYL2* genes encoding xylose reductase (XR) and xylitol dehydrogenase (XDH) and the endogenous *XKS1* gene encoding xylulokinase (XK), stable xylose fermenting *S. cerevisiae* strains have been obtained. However, xylitol is secreted as a result of the difference in cofactor usage between the NAD(P)H-dependent XR and the strictly NAD⁺-dependent XDH steps.

Because of its dual cofactor utilization, XR from *P. stipitis* has been preferred for the construction of xylose-fermenting recombinant *S. cerevisiae* strains. Presently no known XR can reduce xylose to xylitol with NADH as sole cofactor. Among natural xylose-utilising yeasts *Candida utilis* XR exclusively uses NADPH, whereas *C. shehatae, P. segobiensis, P. stipitis* and *Pachysolen tannophilus* XRs use both NADPH and NADH in the reduction of xylose to xylitol. The *C. parapsilosis* XR also uses both NADPH and NADH for xylose reduction, but unlike the other yeasts it prefers NADH. Only yeasts harbouring a NADH-linked xylose reductase activity display significant alcoholic fermentation.

The ethanol yield in recombinant xylose-fermenting *S. cerevisiae* strains is far from the theoretical maximum of 0.51 g g⁻¹, partly because a significant fraction of the consumed xylose is secreted as xylitol. Xylitol formation in *P. tannophilus* has been reduced by addition of hydrogen acceptors. These compounds reoxidized NAD⁺, which is needed in the XDH reaction. Xylitol formation in recombinant *S. cerevisiae* has been reduced by the addition of acetoin, furfural and acetaldehyde. Xylitol formation can also be decreased by shifting the cofactor usage in the XR-step from NADPH to NADH. This was achieved by changing the intracellular pool of NADPH by blocking or reducing the oxidative pentose phosphate pathway (PPP) flux through modification of the glucose 6-phosphate dehydrogenase activity. This resulted in improved ethanol yield at the expense of impaired growth rate on glucose and decreased xylose consumption rate.

The *XYL1* gene has been subjected to site-specific mutagenesis to reduce the XR-affinity for NADPH (Zhang and Lee, 1997, Site-directed mutagenesis of the cystein residues in the Pichia stipitis xylose reductase. FEMS Microbiol Lett 147:227-232, and Kostrzynska et al 1998, Mutational analysis of the role of the conserved lysine 270 in the Pichia stipitis xylose reductase, FEMS Microbiol Lett.159:107-112) Chemical modification studies on XR showed that cysteine and histidine residues might be involved in NADPH binding. However, mutation of three cysteine residues (Cys19, Cys27 and Cys130) to serine enhanced the apparent Km for NADPH less than 4-fold, indicating that none of these cysteine residues were directly involved in NADPH binding. A 17 times lower affinity for NADPH was achieved when the *XYL1* gene carried the K270M (lysine → methionine) mutation, whereas the affinity for NADH remained unchanged. The only difference between NADPH and NADH is the presence of a phosphate group in NADPH, and it was suggested that Lys270 binds to the phosphate group of NADPH.

Attempts have also been made to change the cofactor specificity of XDH towards NADP⁺. A NADP⁺ recognition sequence from *Thermoanaerobium brockii* was introduced in the *XYL2* gene resulting in equal apparent Km values for NAD⁺ and NADP⁺. However, this was achieved at the expense of reduced NAD⁺ specificity combined with unaltered NADP⁺ specificity. The mutated *XYL2* gene mediated xylose growth when co expressed with the *XYL1* gene in *S. cerevisiae* while ethanolic fermentation of xylose was not reported.

### Summary of the present invention

The present invention relates to a novel *Saccharomyces cerevisiae* strains expressing K270M mutated *P. stipitis XYL1* gene (Kostrzynska et al 1998) at two different levels together with the native *P. stipitis XYL2* gene and the overexpressed endogenous *XKS1* gene. The strains were compared with strains: expressing the native *P. stipitis XYL1* gene together with *XYL2* and *XKS1* genes, for xylose consumption and ethanol formation.

### MATERIALS AND METHODS

### Strains.

*Escherichia coli* DH5a (Life Technologies, Rockville, MD, USA) was used for subcloning. All strains were stored in 20% glycerol at -80°C. Yeast cells from freshly streaked YPD plates were used for inoculation. Yeast strains are listed in Table 1.

**TABLE 1. Yeast strains used in the present investigation.**

| Strains | Relevant genotype | Relevant phenotype | Reference |
|---|---|---|---|
| TMB3001 | CEN.PK 113-7A (*MATα his3-Δ1 MAL2-8c SUC2) his3*::YIp XR/XDH/XK | Produces XR, XDH and XK | (1) |
| | | | |
| TMB3260 | TMB3001 *XYL1::*pB3 PGK *XYL1* | Overproduces XR | (2) |
| | | | |
| TMB3265 | CEN.PK 113-11C (*MATα his3-Δ1 ura3-52 MAL2-8c SUC2) his3::* YIpXDH/XK | Produces XDH and XK | (3) |
| | | | |
| TMB3270 | TMB3265 *ura3::*YIplac211 PGK *XYL1*(K270M) | Produces mutated XR | This work |
| | | | |
| TMB3271 | TMB3270 *XYL1::*pB3 PGK *XYL1* (K270M) | Overproduces mutated XR | This work |

### Nucleic acid manipulation.

Plasmid DNA was prepared with a BioRad Plasmid Miniprep Kit (Hercules, CA, USA). Restriction and modification enzymes were obtained from Fermentas (Vilnius, Lithuania). The QIAquick Gel Extraction Kit (QIAGEN GmbH, Hilden, Germany) was used for DNA extractions from agarose.

### Transformation.

Competent cells of *E. coli* DH5α were prepared and transformed by the method of Inoue. Yeast transformation was performed using the lithium acetate method. Yeast cells were incubated overnight in YPD-medium before being streaked out on selective medium. *E. coli* transformants were selected on Luria-Bertani (LB) plates with 100 µg ml⁻¹ ampicillin (IBI Shelton Scientific Inc., Shelton, CT, USA). *S. cerevisiae* transformants were selected on plates with Yeast Nitrogen Base w/o amino-acids (Difco, Sparks, MD) containing 20 g l⁻¹ glucose and 50 mg l⁻¹ uracil or on YPD plates with 100 µg ml⁻¹ zeocin (Invitrogen, Groningen, The Netherlands).

### Construction of a new strain TMB3270.

The *PGK1* promoter and terminator were excised from pB3 PGK using *Nar*I and *Sac*I*,* and inserted in YIplac211 using the same sites, resulting in YIplac211 PGK. Mutated *XYL1* (K270M) was amplified from pSX (K270M) using primers previously described adding *Bam*HI sites to both ends. The *XYL1* PCR product was cut with *Bam*HI and inserted after the *PGK1* promoter at the *Bgl*II site of YIplac211 PGK, resulting in YIplac211 PGK *XYL1* (K270M). The correct orientation was verified by PCR and the correctness of the vector was verified by restriction analysis.

YIplac211 PGK *XYL1* (K270M) was cleaved with *Bpu*10I within the *URA3* gene. The cleavage product was used for transformation of TMB3265 (XDH-XK) resulting in TMB3270. Integration at the correct locus in TMB3270 was verified with PCR, using the primer 5'CAC GGA AAT GTT GAA TAC TCA TAC TC 3' annealing to the YIplac211 PGK *XVL1* (K270M) vector and 5' GTT ACT TGG TTC TGG CGA GGT A 3' annealing downstream of the *URA3* gene in the yeast genome. The K270M mutation was verified by sequencing.

### Construction of a new strain TMB3271.

The *PGK1* promoter and terminator together with the *XYL1* (K270M) gene were excised from Ylplac211 PGK *XYL1* (K270M) using *Nar*I and *Sac*I*.* The fragment was inserted into pB3 PGK after removal of the *PGK1* promoter and terminator with *Nar*I and *Sac*I, resulting in pB3 PGK *XYL1*(K270M). The correctness of the vector was verified by restriction analysis and the K270M mutation was verified by sequencing. The plasmid was cleaved with *Bsh*TI within the *XYL1* gene. The cleaved plasmid was used for integration in the *XYL1* K270M gene of TMB3270. The transformant TMB3271 was selected on YPD-plates with zeocin.

### Batch-fermentation.

Oxygen-limited batch-fermentation of xylose was conducted as previously described (Jeppsson, M., B. Johansson, B. Hahn-Hägerdal, and M. Gorwa Grauslund. 2002. Reduced oxidative pentose phosphate pathway flux in recombinant xylose-utilizing Saccharomyces cerevisiae strains improves the ethanol yield from xylose. Appl. Environ. Microbiol. 68:1604-1609).

### Continuous cultivation.

Cells from an overnight culture were used for inoculation of 1.51 defined mineral medium (30) with 10 g l⁻¹ glucose and 10 g l⁻¹ xylose in a Bioflo-III fermentor (New Brunswick Scientific, Edison, NJ, USA). Antifoam was added at 0.05% (v/v) (Dow Corning® Antifoam RD Emulsion, BDH Laboratory Supplies, Poole, UK). After depletion of glucose, continuous cultivation with 10 g l⁻¹ glucose and 10 g l⁻¹ xylose was set up at dilution rate of 0.06 l⁻¹ at 30°C and pH 5.5 controlled by 3 M KOH. Anaerobic conditions were obtained by sparging the fermentor with 0.2 l min⁻¹ nitrogen (containing less than 5 ppm O₂) as measured with a gas mass flowmeter (Bronkhorst, Ruurlo, The Netherlands) and a stirring speed of 200 rpm was used.

### Analyses.

Substrate and product concentrations, cell dry weight, RNA, proteins and carbohydrates were measured as earlier described (Jeppsson, supra). The previously developed stoichiometric flux model (3) was used to evaluate the cofactor usage by XR.

### Enzyme activities.

Enzyme activities were determined in cells from shake-flask cultures growing exponentially in defined mineral medium (Verduyn, C., Postma, E., Scheffers, W. A. and Van Dijken, J. P. 1992. Effect of benzoic acid on metabolic fluxes in yeasts: a continuous-culture study on the regulation of respiration and alcoholic fermentation. Yeast. 8: 501-517) with 20g l⁻¹ glucose as carbon source. Precultures were prepared under the same conditions. Crude extracts were obtained using the YPER reagent (Pierce, Rockford, IL, USA). The protein concentration was determined using the Coomassie protein assay reagent (Pierce) with bovine serum albumine as a standard. XR-activity was determined as previously described (Eliasson, A., C. Christensson, C. F. Wahlbom, and B. Hahn-Hägerdal. 2000, Anaerobic xylose fermentation by recombinant Saccharomyces cerevisiae carrying XYL1, XYL2, and XKS1 in mineral medium chemostat cultures Appl. Environ. Microbiol. 66:3381-3386.) except for the strains carrying the mutated XR, where 1.05M xylose and 0.3mM NADPH were used.

### RESULTS

### Effect of the XYL1 K270M mutation on xylose fermentation.

Kostrzynska *et al.* (1998) subjected the *P. stipitis* XR to site-specific mutagenesis in order to modify the affinity for NADPH. The mutation at amino acid position 270 resulted in an apparent Km of 185 µM for NADPH, which is 17 times higher than the apparent Km for NADPH of the native XR (11 µM). However, the mutation in XR K270M also enhanced the apparent Km for xylose 15 fold (625mM vs. 43mM), whereas the apparent Km for NADH was not influenced (31µM vs. 27µM).

The *XYL1* K270M gene was introduced in strain *S. cerevisiae* TMB3265 already harboring the *P. stipitis XYL2* and the endogenously overexpressed *XKS1* genes, resulting in strain TMB3270. The NADPH- and NADH-dependent XR activities were 0.74 and 0.63 U mg⁻¹, respectively, in TMB3270 (Table 2). The control strain TMB3001, which harbours the native *XYL1* gene together with the *XYL2* and *XKS1* genes, had NADPH- and NADH-dependent activities of 0.48 and 0.31 U mg⁻¹, respectively.

**TABLE 2. Specific XR-activity (U mg protein⁻¹) for TMB3001, TMB3260, TMB3270 and TMB3271. Data shown are average values from two measurements with less than 10% variation.**

| Strains | XR (U/mg) | NADH |
|---|---|---|
| | NADPH | |
| TMB3001 | 0.48 | 0.31 |
| (1 copy of *XYL1*) | | |
| TMB3260 | 3.11 | 1.63 |
| (2 copies of *XYL1*) | | |
| TMB3270 | 0.74 | 0.63 |
| (1 copy of *XYL1* (K270M)) | | |
| TMB3271 | 4.04 | 1.50 |
| (2 copies of *XYL1* (K270M)) | | |

Product formation from xylose was analyzed in batch fermentation with glucose-grown resting cells utilizing xylose under oxygen-limited conditions (Table 3). Very little biomass is formed under these conditions. TMB3270 (*XYL1* K270M) had similar xylose consumption rate (0.155 g biomass⁻¹ h⁻¹) as TMB3001 (0.145 g biomass⁻¹ h⁻¹). The ethanol yield (0.36 g g⁻¹) was enhanced and the xylitol yield (0.17 g g⁻¹) was reduced in TMB3270 compared to the control strain TMB3001 (0.31 g ethanol g xylose⁻¹, 0.29 g xylitol g xylose⁻¹). Acetate and glycerol yields were low, but higher in TMB3270 (0.035 g g⁻¹, 0.072 g g⁻¹) than in TMB3001 (0.025 g g⁻¹, 0.052 g g⁻¹)_{.}

TMB3270, harboring the mutated XR, consumed xylose similarly as the control strain TMB3001 in batch culture with 50g/l xylose. However, the lower xylose affinity of the mutated XR could reduce the xylose consumption rate at low xylose concentrations. The mutation might also influence product formation during co-fermentation of glucose and xylose. Therefore anaerobic continuous cultivation was conducted with TMB3001 and TMB3270 at 10 g l⁻¹ glucose and 10 g l⁻¹ xylose (Table 4). TMB3270 (*XYL1* K270M) had a 58% lower xylose consumption rate than the control strain TMB3001. Ethanol and CO₂ yields were about 10% higher in TMB3270 (0.40 and 0.48 g g substrate⁻¹) than in TMB3001 (0.37 and 0.43 g · g substrate⁻¹). TMB3270 had a reduced xylitol yield per consumed xylose (0.31 g . g xylose⁻¹) compared to TMB3001 (0.52 g . g xylose⁻¹), which agrees with the results from the batch fermentation (Table 3). TMB3270 also had a lower glycerol yield (0.084 g . g substrate⁻¹) than TMB3001 (0.095 g . g substrate⁻¹), whereas the acetate yield was slightly higher in TMB3270 (0.002 g . g substrate⁻¹) compared to TMB3001 (0.001 g. g substrate⁻¹). The biomass yields were similar in the two strains (0.094 and 0.095 g . g substrate⁻¹). The biomass formation was 1.1 and 1.0 g l⁻¹ in TMB3001 and TMB3270, respectively.

**TABLE 3. Effect of decreased XR-affinity for NADPH on xylose fermentation by recombinant S. cerevisiae in oxygen-limited batches with 50 g l⁻¹ xylose as sole carbon source. Specific xylose consumption rate (g x g biomass⁻¹ x h⁻¹) and ethanol, xylitol, acetate and glycerol yields (g x g consumed xylose⁻¹) and carbon recoveries (cmoles out (cmoles in)⁻¹) were determined after 60-70 hours. The presented values are the average of two independent fermentations experiments plus/minus the deviation from the average. In calculation of carbon recovery, 0.5 moles of CO₂ are assumed to be formed for each cmole of ethanol or acetate produced.**

| | Xylose | | | | | |
|---|---|---|---|---|---|---|
| Strains | Consumption | Y | Y | Y | Y | C- |
| | Rate | Ethanol | Xylitol | Acetate | Glycerol | recovery |
| | | | | | | |
| TMB3001¹ (1 copy of XYL1) | 0.145 ± 0.002 | 0.31 ± 0.01 | 0.29 ± 0.01 | 0.025 ± 0.001 | 0.052 ± 0.004 | 0.98 |
| | | | | | | |
| TMB3260² (2 copies of XYL1) | 0.245 ± 0.005 | 0.30 ± 0.01 | 0.13 ± 0.01 | 0.046 ± 0.007 | 0.161 ± 0.001 | 0.95 |
| | | | | | | |
| TMB3270 (1 copy of XYL1 (K270M)) | 0.155 ± 0.005 | 0.36 ± 0.01 | 0.17± 0.01 | 0.035± 0.001 | 0.072 ± 0.005 | 0.99 |
| | | | | | | |
| TMB3271 (2 copies of XYL1 (K270M)) | 0.226 ± 0.020 | 0.31 ± 0.01 | 0.09 ± 0.01 | 0.060 ± 0.008 | 0.181 ± 0.014 | 0.97 |

| | | | | | | |
|---|---|---|---|---|---|---|
| Fermentation data from ¹(12) and ²(14). | | | | | | |

Effect of enhanced expression of *XYL1* K270M on xylose fermentation. TMB3001 has been shown to use both NADPH and NADH in the XR-reaction. Product formation in batch (Table 3) and continuous cultivation (Table 4) indicated that the increased Km value for NADPH of the mutated XR in TMB3270 resulted in reduced NADPH-usage in the XR-step. Since the NADH-dependent activity was similar in the two strains (Table 2), a too low XR-activity might explain the lower xylose consumption in TMB3270. It has previously been shown that the XR activity limited the xylose consumption rate in TMB3001.

**TABLE 4. Continuous cultivation of TMB3001, TMB3270, TMB3260 and TMB3271 under anaerobic conditions at a dilution rate of 0.06h⁻¹ with 10g l⁻¹ glucose and 10g l⁻¹ xylose as carbon-source. Consumption rates (g g biomass⁻¹ h⁻¹), yields (g g consumed substrate⁻¹) and c-recovery are shown. Steady-state was assumed after at least 6 fermentor-volumes had passed the fermentor.**

| Strains | Glucose | Xylose | Y | Y | Y | Y | Y | Y | Y | C- |
|---|---|---|---|---|---|---|---|---|---|---|
| | Cons. rate | Cons. rate | Ethanol | Xylitol | Xylitol* | Acetate | Glycerol | CO₂ | v | recovery |
| TMB3001 | 0.52 | 0.12 | 0.37 | 0.10 | 0.52 | 0.001 | 0.095 | 0.43 | 0.094 | 1.08 |
| (1 copy of *XYL1*) | | | | | | | | | | |
| | | | | | | | | | | |
| TMB3260 | 0.51 | 0.19 | 0.36 | 0.16 | 0.58 | 0.004 | 0.107 | 0.39 | 0.085 | 1.09 |
| (2 copies of *XYL1*) | | | | | | | | | | |
| | | | | | | | | | | |
| TMB3270 | 0.58 | 0.05 | 0.40 | 0.02 | 0.31 | 0.002 | 0.084 | 0.48 | 0.095 | 1.07 |
| (1 copy of *XYL1* (K270M)) | | | | | | | | | | |
| | | | | | | | | | | |
| TMB3271 | 0.45 | 0.16 | 0.40 | 0.12 | 0.44 | 0.003 | 0.053 | 0.40 | 0.098 | 1.07 |
| (2 copies of *XYL1* (K270M)) | | | | | | | | | | |

| | | | | | | | | | | |
|---|---|---|---|---|---|---|---|---|---|---|
| *Xylitol yield on xylose | | | | | | | | | | |

Another copy of the mutated *XYL1* (K270M) gene was integrated in TMB3270, resulting in TMB3271. The NADPH and NADH dependent XR-activity in TMB3271 was 4.04 and 1.50 U mg⁻¹, respectively, which is 2-5 times higher than in TMB3270 (Table 2). The high XR-activity strain TMB3260 was included as control strain for TMB3271 in this investigation. TMB3260 harbours two copies of native the *XYL1* gene together with the *XYL2* and *XKS1* genes, and had NADPH- and NADH-dependent activities of 3.11 and 1.63 U mg⁻¹, respectively (Table 2).

Xylose consumption and product formation with TMB3271 (2 copies of *XYL1* (K270M)) were compared with TMB3270 (1 copy of *XYL1* (K270M)), TMB3001 (1 copy of *XYL1*) and TMB3260 (2 copies of *XYL1*) in oxygen-limited batch fermentation using resting cells (Table 3). There was no significant difference between TMB3260 and TMB3271 expressing high levels of *XYL1* and *XYL1* (K270M), respectively. Both had higher xylose consumption rate, decreased xylitol yield, enhanced glycerol and acetate yields, and similar ethanol yield compared to TMB3001.

Product formation with TMB3260 and TMB3271, carrying 2 copies of the *XYL1* and *XYL1* (K270M) genes, respectively, was also determined in anaerobic continuous culture with 10g l⁻¹ glucose and 10g l⁻¹ xylose as carbon sources (Table 4). The enhanced XR-activity resulted in 58% and 220% higher xylose uptake in TMB3260 and TMB3271 (*XYL1* K270M), compared to their respective low XR-activity strains TMB3001 and TMB3270 *(XYL1* K270M). The K270M mutation in TMB3271 resulted in higher ethanol- and CO₂-yield (0.40 g g⁻¹ and 0.40 g g⁻¹) than for the control strain TMB3260 (0.36 g g⁻¹ and 0.39 g g⁻¹), which agrees with the enhanced ethanol- and CO₂-yields found at low XR-activity in TMB3270 (Table 4). The xylitol yield was lower in TMB3271 (0.44 g xylitol g xylose⁻¹) than in TMB3260 (0.58 g xylitol g xylose⁻¹). The biomass yield was higher in TMB3271 (0.098 g . g substrate⁻¹), than in TMB3260 (0.85 g . g substrate⁻¹). The biomass formation was 1.1 and 1.2 g l⁻¹ in TMB3260 and TMB3271, respectively. TMB3271 had a 2-fold decrease in glycerol yield and a slight decrease in acetate yield compared to TMB3260.

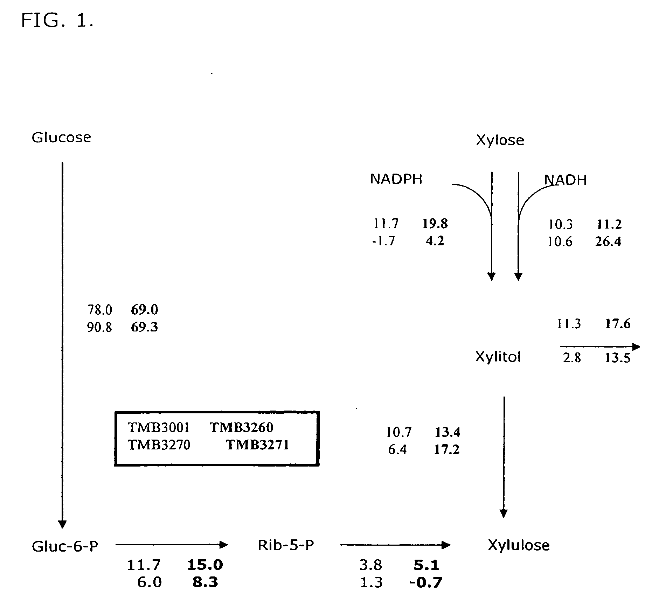

**Flux analysis.** A stoichiometric flux model (3) was used to elucidate the cofactor usage by XR in strains harboring native *XYL1* (TMB3001 and TMB3260) and mutated *XYL1* K270M (TMB3270 and TMB3271), respectively. The model showed that strains expressing mutated XR used a greater fraction of NADH in the XR-reaction than strains expressing native XR (Figure 1). According to the model, the XR-reaction in TMB3001 used 47% NADH, whereas the XR-reaction in TMB3270 (K270M) used only NADH for the reduction. In strains with higher XR-activity, TMB3260 and TMB3271 (*XYL1* K270M), 36 and 86% NADH, respectively, was used in the XR-step. The oxidative pentose phosphate pathway is a major source of NADPH in yeast, and more glucose channeled through this pathway in TMB3001 (15%) and TMB3260 (22%) than in the mutant-carrying TMB3270 (7%) and TMB3271 (12%). As a result less carbon dioxide was formed in the mutant-carrying strains.

It has previously been shown that XR reduced dihydroxyacetone phosphate (DHAP) with NADPH. The flux model, however, assumed a strictly NADH-dependent DHAP-reduction. When the reaction was changed in the model to use 50% NADH and 50% NADPH for reduction of DHAP, the NADH usage in the XR-reaction increased for all strains, and it was higher in the strains carrying the mutated XR. The strains carrying the mutated XR (TMB3270 and TMB3271) also had a lower oxidative PPP flux in the changed model.

By using a mutated XR with decreased affinity for NADPH, we aimed at shifting product formation from xylitol to ethanol in recombinant *S. cerevisiae.* TMB3270 and TMB3271 harboring the mutated XR showed lower xylitol and glycerol yields, accompanied with higher ethanol and CO₂ yields compared to strains with native XR. The significantly reduced anaerobic glycerol yield in TMB3271 most likely resulted from the reduced demand for NAD⁺ reoxidation when the XR-reaction mainly uses NADH. A metabolic flux model demonstrated that the strains harboring the mutated XR used a larger fraction of NADH in the reduction of xylose. The enhanced biomass yield fits with the decreased glycerol yield in TMB3271, since NAD⁺ is consumed during biomass formation and regenerated during glycerol formation.

Several approaches have been taken to improve ethanol yield and productivity by changing the redox metabolism during xylose fermentation in recombinant strains of *S*. *cerevisiae* (Table 5). Enhanced ethanol and decreased xylitol yields (30 and 70%, respectively) were observed in TMB3255 disrupted in the oxidative PPP gene *ZWF1,* as a result of less NADPH being available for the XR-reaction (4). The overproduction of transhydrogenase from *Azotobacter vinelandii,* converting NAD⁺ and NADPH into NADH and NADP⁺ in *S. cerevisiae,* was also likely to reduce the intracellular NADPH concentration. The TH-overproducing strain showed a slight decrease in xylitol yield whereas no change was observed in ethanol yield (5).

Expression of a NADP⁺ dependent glyceraldehyde-3-phosphate dehydrogenase (GAPDH) in *S. cerevisiae* enhanced the ethanol yield from xylose by 25% (Verho et al. 2003. Engineering of redox cofactor metabolism for improved pentose fermentation in Saccharomyces cerevisiae. Appl Environ Microbiol 69: 5892-7)The modification was suggested to result in less oxidative PPP flux and hence lower CO₂ production. The ethanol yield was enhanced by 127% when the oxidative PPP gene *ZWF1* was disrupted in combination with GAPDH over-production. However, the ethanol yield was not higher than in the other studies due to low initial yield of the control strain.

**TABLE 5. Xylose consumption rate (g g biomass⁻¹ h⁻¹), ethanol yield (g g sugar⁻¹) and xylitol yield (g g xylose⁻¹) in fermentation with recombinant S. cerevisiae strains engineered to enhance ethanol yield from xylose. The relevant genotype and phenotype is reported, as well as oxygenation, O₂ (Anaerobic (-), Oxygen-limitation (+/-)), Fermentation mode (Continuous cultivation (C), Batch cultivation (B)) and glucose (Glu) and xylose (Xyl) concentrations in g l⁻¹.**

| Strain | Relevant genotype / | O₂ | Mode | D | Glu | Xyl | Xylose | Y_{Ethanol} | Y_{Xylitol} | Reference | Reference |
|---|---|---|---|---|---|---|---|---|---|---|---|
| | phenotype | | | | | | consumption | (% increase) | | Strain | Ferm.data |
| | | | | | | | rate | | | | |
| TMB3001 | 1 copy of *XYL1* | - | C | 0.06 | 10 | 10 | 0.12 | 0.37 | 0.52 | (1) | This work |
| TMB3260 | 2 copies of *XYL1* | - | C | 0.06 | 10 | 10 | 0.19 | 0.36 | 0.58 | (2) | This work |
| TMB3270 | 1 copy of *XYL1* (K270M) | - | C | 0.06 | 10 | 10 | 0.05 | 0.40 (8%) | 0.31 | This work | This work |
| | | | | | | | | | | | |
| TMB3271 | 2 copies of *XYL1* (K270M) | - | C | 0.06 | 10 | 10 | 0.16 | 0.40 (11%) | 0.44 | This work | This work |
| | | | | | | | | | | | |
| TMB3001 | | - | C | 0.06 | 20 | 20 | 0.23 | 0.30 | 0.43 | (1) | (4) |
| TMB3255 | Δ*zwf1* | - | C | 0.06 | 20 | 20 | 0.12 | 0.39 (30%) | 0.13 | (4) | (4) |
| TMB3001 | | +/- | B | - | - | 50 | 0.15 | 0.31 | 0.29 | (1) | (4) |
| TMB3253 | Control | +/- | B | - | - | 50 | 0.16 | 0.28 | 0.34 | (5) | (5) |
| TMB3254 | TH | +/- | B | - | - | 50 | 0.16 | 0.28 | 0.30 | (5) | (5) |
| | | | | | | | | | | | |
| H2674 | Control | - | B | - | - | 50 | 0.07* | 0.14 | 0.56 | (6) | (6) |
| H2673 | GDP1 | - | B | - | - | 50 | 0.06* | 0.17 (25%) | 0.49 | (6) | (6) |
| H2684 | *GDP1 Δzwf1* | - | B | - | - | 50 | 0.06* | 0.31 (127%) | 0.35 | (6) | (6) |
| TMB3001 | | - | C | 0.05 | 20 | 50 | 0.31 | 0.28 | 0.56 | (1) | (7) |
| CBP.CR4 | Δ*gdh1 GDH2* | - | C | 0.05 | 20 | 50 | 0.31 | 0.34 (19%) | 0.47 | (7) | (7) |
| CBP.CR5 | Δ*gdh1 GS-GOGAT* | - | C | 0.05 | 20 | 50 | 0.34 | 0.33 (16%) | 0.44 | (7) | (7) |

| | | | | | | | | | | | |
|---|---|---|---|---|---|---|---|---|---|---|---|
| *Assumed 120h cultivation time (only approximate cultivation time was reported in (31)). | | | | | | | | | | | |

An enhanced ethanol yield on xylose was also obtained by metabolic engineering of the ammonium assimilation (7). The ethanol yield increased by 19%, and the xylitol yield decreased by 16%, by deleting the *GDH1* gene, encoding a NADPH dependent glutamate dehydrogenase, and by overexpressing the *GDH2* gene, encoding a NADH dependent glutamate dehydrogenase. Another strategy was the overexpression of *GLT1* and *GLN1* genes, encoding the NADH and ATP requiring GS-GOGAT complex, in the *GDH1*-deleted strain, which resulted in 16% higher ethanol yield.

This investigation shows that it is possible to improve the ethanol yield (11% higher in TMB3271 than in TMB3260) at maintained xylose consumption rate by expressing the *XYL1* K270M mutant at high level. The cultivation conditions were not the same in the different investigations, but since TMB3001 has been included in all cases except for the *GDP1*-overexpressing strains, it is clear that TMB3260 (2 copies of *XYL1*), TMB3271 (2 copies of *XYL1* (K270M)) and CBP.CR5 (Δ*gdh1 GS-GOGAT*), have comparably high xylose consumption rates (Table 5). The CBP.CR5 (*Δgdh1 GS-GOGAT*) and TMB3271 (2 copies of *XYL1* (K270M) strains also had high ethanol yields compared to TMB3001. The metabolic engineering results of the different studies demonstrate how difficult it is to enhance the ethanol yield by interfering with the redox metabolism, and raise the question of the flexibility limit of the central metabolic pathways in *S. cerevisiae.*

### FIGURE LEGEND

**Figure 1****.** Fluxes in xylose pathway (mmol * g biomass⁻¹ * h⁻¹) for TMB3001 (upper value, not bold), TMB3270 (lower value, not bold), TMB3260 (higher value, bold) and TMB3271 (lower value, bold) cultivated in continuous culture with 10 g l⁻¹ glucose and 10 g l⁻¹ xylose. Sugar uptake (glucose + xylose) was normalized to 100 mmol * g biomass⁻¹ * h⁻¹.

### REFERENCES

1. Eliasson, A., C. Christensson, C. F. Wahlbom, and B. Hahn-Hägerdal. 2000. Anaerobic xylose fermentation by recombinant Saccharomyces cerevisiae carrying XYL1, XYL2, and XKS1 in mineral medium chemostat cultures. Appl. Environ. Microbiol. 66:3381-3386.
2. Jeppsson, M., K. Träff, B. Johansson, B. Hahn-Hägerdal, and M.-F. Gorwa-Grauslund. 2003. Effect of enhanced xylose reductase activity on xylose consumption and product distribution in xylose-fermenting recombinant. Saccharomyces cerevisiae. FEMS Yeast Res. 3:167-175.
3. Träff-Bjerre, K. L., M. Jeppsson, B. Hahn-Hägerdal, and M.-F. Gorwa-Grauslund. 2004. Endogeneous NADPH-dependent aldose reductase activity influences product formation during xylose consumption in recombinant Saccharomyces cerevisiae. Yeast 21:141-150.
4. Jeppsson, M., B. Johansson, B. Hahn-Hägerdal, and M. Gorwa-Grauslund. 2002. Reduced oxidative pentose phosphate pathway flux in recombinant xylose-utilizing Saccharomyces cerevisiae strains improves the ethanol yield from xylose. Appl. Environ. Microbiol. 68:1604-1609.
5. Jeppsson, M., B. Johansson, P. Ruhdal-Jensen, B. Hahn-Hägerdal, and M. Gorwa-Grauslund. 2003. The level of glucose-6-phosphate dehydrogenase activity strongly influences xylose fermentation and inhibitor sensitivity in recombinant Saccharomyces cerevisiae strains. Yeast 20:1263-1272.
6. Zhang, Y., and H. Lee. 1997. Site-directed mutagenesis of the cysteine residues in the Pichia stipitis xylose reductase. FEMS Microbiol. Lett. 147:227-232.
7. Roca, C., J. Nielsen, and L. Olsson. 2003. Metabolic engineering of ammonium assimilation in xylose-fermenting Saccharomyces cerevisiae improves ethanol production. Appl. Environ. Microbiol. 69:4732-4736.

## Claims

1. A xylose-utilizing *Saccharomyces cerevisiae* mutant strain fermenting xylose to ethanol wherein the strain expresses
a) two copies of a K270M mutated *P. stipitis XYL1* gene,
b) the native *P. stipitis XYL2* gene and
c) overexpresses the *XKS1* gene endogenous to *Saccharomyces cerevisiae.*

## Patentansprüche

1. Ein Xylose verwertender, mutierter *Saccharomyces cerevisiae*-Stamm, der Xylose zu Ethanol fermentiert, wobei der Stamm
a) zwei Kopien eines K270M mutierten *P. stipitis XYL1*-Gens exprimiert,
b) das native *P. stipitis XYL2*-Gen exprimiert und
c) das *Saccharomyces cerevisiae* endogene XKSI-Gen überexprimiert.

## Revendications

1. Xylose utilisant la souche mutée *Saccharomyces cerevisiae* produisant de la fermentation de xylose en éthanol, dans lequel la souche exprime
a) deux copies d'un gène *P. stipitis XYL1* muté K270M,
b) le gène natif *P. stipitis XYL2,* et
c) surexprime le gène *XKS1* endogène en *Saccharomyces cerevisiae.*
